# EUROPÄISCHE PATENTSCHRIFT

(11) **EP 1 844 033 B1**
(45) Veröffentlichungstag und Bekanntmachung des Hinweises auf die Patenterteilung: **27.07.2011**
(21) Anmeldenummer: 06707815.4
(22) Anmeldetag: 24.01.2006
(51) Int. Cl.: C07D 311/76

(54) **VERFAHREN ZUR HERSTELLUNG VON ISOCHROMAN UND DERIVATEN DAVON**
METHOD FOR PRODUCING ISOCHROMANE AND DERIVATIVES THEREOF
PROCÉDÉ DE PRODUCTION D'ISOCHROMANE ET DE SES DÉRIVÉS

(30) Priorität: 28.01.2005 DE 102005004021; 08.02.2005 DE 102005005620
(43) Veröffentlichungstag der Anmeldung: 17.10.2007
(73) Patentinhaber: Boehringer Ingelheim Pharma GmbH & Co. KG, 55216 Ingelheim am Rhein (DE)
(72) Erfinder: LOCK, Ralf, 55122 Mainz Am Rhein (DE)
(74) Vertreter: Hammann, Heinz
(86) Internationale Anmeldenummer: PCT/EP2006/050401
(87) Internationale Veröffentlichungsnummer: WO 2006/079622

(56) Entgegenhaltungen:
- DE-A1- 2 000 339
- US-A- 4 031 219
- DE SILVA, S. O. ET AL: "A convergent route to phthalideisoquinoline alkaloids via directed metalation of tertiary benzamides" CANADIAN JOURNAL OF CHEMISTRY , 57(13), 1598-605 CODEN: CJCHAG; ISSN: 0008-4042, 1979, XP009070692
- MARIE K. GOO-ON, LOUIS H. SCHWARTZMAN, AND G. FORREST WOODS: "SYNTHESIS OF SOME 3,3-DIMETHYL-1-AMINOINDANS" JOURNAL OF ORGANIC CHEMISTRY, Bd. 19, Nr. 3, 1956, Seiten 305-311, XP002393823

## Beschreibung

Isochroman und seine Derivate der allgemeinen Formel 1, worin R¹, R² und R³ jeweils unabhängig voneinander die Bedeutung von C₁₋₆-Alkyl, C₂₋₆-Alkenyl, C₂₋₆-Alkinyl oder Aryl und R⁴ die Bedeutung von H, OC₁₋₆-Alkyl, OAryl, OH, C₁₋₆-Alkyl, Halogen, CN oder NO₂ hat, 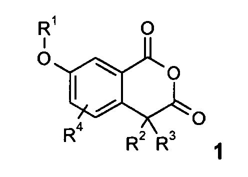 sind wertvolle Zwischenprodukte zur Synthese pharmazeutischer Wirkstoffe wie z.B. Gliquidon bzw. Glurenorm, welches zu den pharmazeutisch als Mittel zur Behandlung der Altersdiabetes gehört und von hohem kommerziellem Interesse sind. Die Herstellung von Verbindungen der Formel 1 wird in der DE 2000339 beschrieben.

### BESCHREIBUNG DER ERFINDUNG

Die vorliegende Erfindung betrifft ein Verfahren zur Herstellung einer Verbindung der Formel **1**, 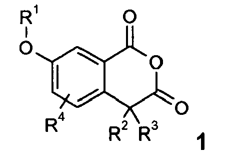 worin R¹, R² und R³ jeweils unabhängig voneinander die Bedeutung von C₁₋₆-Alkyl, C₂₋₆-Alkenyl, C₂₋₆-Alkinyl oder Aryl und R⁴ die Bedeutung von H, OC₁₋₆-Alkyl, OAryl, OH, C₁₋₆-Alkyl, Halogen, CN oder NO₂, bevorzugt H, C₁₋₆-Alkyl, Halogen, CN oder NO₂ hat, dadurch gekennzeichnet, dass man
a) Ein Acrylsäurederivat der Formel **4** mit einer HBr-Lösung behandelt 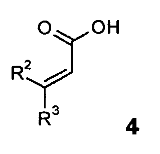 und anschließend mit einer Verbindung der Formel **5** in Gegenwart einer Lewis-Säure umsetzt; 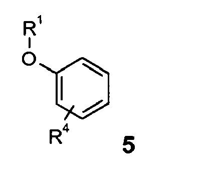
b) die daraus erhaltene Verbindung **3**, 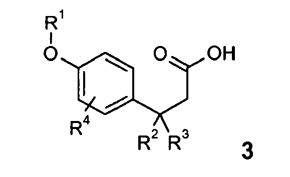 mit Polyphosphorsäure, Mischungen aus Phosphorpentoxid und Methansulfonsäure oder einer Lewis-Säure wie zum Bsp. Wismut- oder einem Lanthanidtriflat oder-bistrifluormethansulfonylimid, bevorzugt Polyphosphorsäure behandelt oder die Verbindung **3** zunächst in ein Säurehalogenid oder -anhydrid überführt und anschließend mit einer Lewis Säure behandelt und anschließend mit einem Cₗ-₆-Alkylnitrit, bevorzugt Isoamylnitrit umsetzt
c) die daraus erhaltene Verbindung **2**, 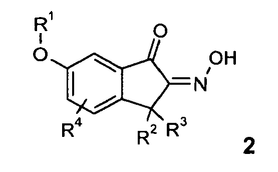 in Gegenwart einer Base mit einem Sulfonsäurehalogenid, bevorzugt Toluolsulfonsäurechlorid umsetzt, verseift und anschließend hydrolysiert.

Bevorzugt ist das obige Verfahren worin R¹, R² und R³ jeweils unabhängig voneinander die Bedeutung von C₁₋₆-Alkyl, besonders bevorzugt C₁₋₄-Akyl hat.

Besonders bevorzugt ist das obige Verfahren worin R¹ R² und R³ jeweils unabhängig voneinander Methyl, Ethyl, *iso*-Propyl oder *tert*-Butyl, besonders bevorzugt Methyl bedeuten.

Bevorzugt ist das obige Verfahren worin R⁴ die Bedeutung von H, Methyl, Ethyl, *iso-*Propyl oder *tert*-Butyl, besonders bevorzugt H hat.

Bevorzugt ist das obige Verfahren worin in Schritt a)
- die Acrylsäure der Formel **4** mit einer 40-80%-iger HBr-Lösung behandelt wird,
- die Mischung für 20-100 min bei einer Temperatur von 0-50°C gerührt wird;
- die Mischung mit einem Lösungsmittel extrahiert wird;
- das Lösungsmittel weitestgehend oder ganz abgezogen wird;
- das Zwischenprodukt in einer Verbindung der Formel 5 mit oder ohne Zugabe eines weiteren Lösungsmittel gelöst wird;
- die Mischung nach Zugabe von Aluminiumchlorid für 2-6 Stunden bei 0-50°C gerührt wird;
- nach Beenden der Reaktion das Produkt durch Extraktion und Umkristallisieren gereinigt wird.

Besonders bevorzugt ist das obige Verfahren worin in Schritt a)
- die Acrylsäure der Formel **4** mit einer 60-65%-iger HBr-Lösung behandelt wird,
- die Mischung für 25-70 min bei einer Temperatur von 20-35°C gerührt wird;
- die Mischung mit einem aromatischen Lösungsmittel extrahiert wird;
- das Lösungsmittel weitestgehend oder ganz abgezogen wird;
- das Zwischenprodukt in einer Verbindung der Formel **5** gelöst wird;
- die Mischung nach Zugabe von Aluminiumchlorid für 3-5 Stunden bei 20-30°C gerührt wird;
- die Reaktion durch Zugabe von Wasser beendet wird;
- das Produkt aus der organischen Phase mit der Lösung einer starken Base extrahiert wird,
- das Lösungsmittel der organischen Phase weitestgehend oder ganz abgezogen wird;
- der Rückstand aus einem aliphatischen Kohlenwasserstoff umkristallisiert wird;

Bevorzugt ist das obige Verfahren worin in Schritt b)
- die erhaltenen Verbindung **3**, mit Polyphosphorsäure vermischt wird;
- die Mischung für 10-50 min auf 50-90°C erwärmt wird;
- die Mischung mit einem Lösungsmittel, bevorzugt ein Ether, Ester, Halogenkohlenwasserstoff oder aromatischer Kohlenwasserstoff, extrahiert wird
- die organische Phase gewaschen wird;
- das Lösungsmittel weitestgehend oder ganz abgezogen wird;
- der Rückstand in einem Alkohol gelöst und mit konz. Salzsäure versetzt wird;
- zu der Mischung ein C₁₋₆-Alklynirit, bevorzugt Isoamylnitrit, gegeben wird;
- die Mischung für 30-90 min bei 30-70°C gerührt wird;
- der entstandene Feststoff abfiltriert und gewaschen wird.

Besonders bevorzugt ist das obige Verfahren worin in Schritt b)
- die erhaltenen Verbindung **3**, mit Polyphosphorsäure vermischt wird;
- die Mischung für 20-40 min auf 60-80°C erwärmt wird;
- zu der Mischung Wasser gegeben wird;
- die Mischung mit einem aromatischen Lösungsmittel extrahiert wird
- die organische Phase mit der Lösung einer starken Base bei pH 8.5-9.5 gewaschen wird;
- die organische Phase mit verdünnter Salzsäure gewaschen wird;
- das Lösungsmittel der organischen Phase weitestgehend oder ganz abgezogen wird;
- der Rückstand in einem Alkohol gelöst und mit konz. Salzsäure versetzt wird;
- zu der Mischung Isoamylnitrit gegeben wird;
- die Mischung für 50-70 min bei 40-60°C gerührt wird;
- der entstandene Feststoff filtriert und gewaschen wird.

Bevorzugt ist das obige Verfahren worin in Schritt c)
- die erhaltene Verbindung **2** in Wasser suspendiert wird;
- die Lösung einer starken Base zugegeben wird;
- die Mischung auf 30-70°C erwärmt wird;
- zu der Mischung Benzolsulfonylchlorid gegeben wird;
- die Mischung für 30-90 min gerührt wird;
- die Mischung mit einem aromatischen Lösungsmittel gewaschen wird;
- die Mischung mit einer Säure angesäuert wird;
- die Mischung mit einem aromatischen Lösungsmittel extrahiert wird;
- das Lösungsmittel der organischen Phase weitestgehend oder ganz abgezogen wird;
- der Rückstand aus einem C₁₋₈-Alkohol umkristallisiert wird.

Besonders bevorzugt ist das obige Verfahren worin in Schritt c)
- die erhaltenen Verbindung **2** in Wasser suspendiert wird;
- die Lösung einer starken Base zugegeben wird;
- die Mischung auf 40-60°C erwärmt wird;
- zu der Mischung Benzolsulfonylchlorid gegeben wird;
- die Mischung für 50-70 min gerührt wird;
- die Mischung mit einem aromatischen Lösungsmittel gewaschen wird;
- die Mischung angesäuert wird mit konz. Salzsäure;
- die Mischung mit einem aromatischen Lösungsmittel extrahiert wird;
- das Lösungsmittel weitestgehend oder ganz abgezogen wird;
- der Rückstand aus einem C₁₋₈-Alkohol umkristallisiert wird.

Insbesondere bevorzugt ist das obige Verfahren zur Herstellung von Isochroman der Formel **1A**, worin 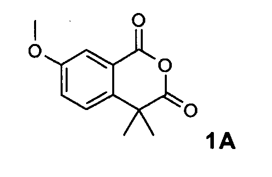
a) folgende Schritte durchgeführt werden:
   - Vermischen von 3,3-Dimethylacrylsäure unter Rühren mit einem Überschuss 62%iger Bromwasserstoffsäure bei 30-50°C;
   - Rühren für 50-70 min bei 15°-40°C;
   - Extrahieren mit Toluol;
   - Einengen im Vakuum zur Trockene;
   - Lösen des Rohprodukts in einem Überschuss Anisol;
   - Zugabe der Mischung zu Anisol und dem 1-2-fachen molaren Überschuss von Aluminiumchlorid, bezogen auf die 3,3-Dimethyacrylsäure;
   - Rühren für 3-5 h bei Raumtemperatur
   - Zugabe der Lösung zu einem Überschuss Eiswasser
   - Rühren für 70-90 min;
   - Extrahieren der organischen Phase bei 50-60°C mit 62%iger Bromwasserstoffsäure, mit 1-2M Natronlauge, diese Natronlauge-Phase zweimal mit Toluol und anschließend mit Bromwasserstoffsäure ansäuern und weitere zweimal mit Toluol;
   - Einengen der organischen Phase zur Trockene;
   - Umkristallisieren des Rückstandes aus Methylcyclohexan;
   - Trocknen des Rückstandes im Vakuum bei 40-60°C;
b) Umsetzen der so erhaltenen Verbindung **3A**, wobei folgende Schritte durchgeführt werden: 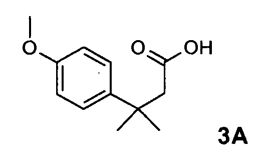
   - Zugabe der Verbindung **3** zu einem Überschuss Polyphosphorsäure;
   - Erwärmen der Mischung auf 60-80°C erwärmt;
   - Rühren der Mischung für 20-40min;
   - Abkühlen auf 40-60°C;
   - Zugabe eines Überschuss Wasser unter Kühlung;
   - Extraktion der Mischung mit Toluol;
   - Waschen mit Natronlauge bei pH 8.5-9.5;
   - Extrahieren der wässrigen Phase mit Toluol;
   - Waschen der vereinigten Toluolphasen mit verdünnter Salzsäure;
   - Einengen im Vakuum;
   - Lösen des Rückstandes in einem Überschuss einer Mischung bestehend aus 4.5 bis 5.5 Volumenteilen Methanol und einem Volumenteil konz. Salzäure;
   - Erwärmen auf 40-60°C;
   - Zugabe von einem 2.5 bis 3 fachen molaren Überschuss an Isoamylnitrit, bezogen auf die Verbindung der Formel **3**;
   - Rühren für 50-70 min bei 40-60°C;
   - Abkühlen und Filtrieren des Feststoffs;
   - Waschen mit Methanol;
   - Trocknen des Rückstandes im Vakuum bei 40-60°C;
c) Umsetzen der so erhaltenen Verbindung **2A**, wobei folgende Schritte durchgeführt werden: 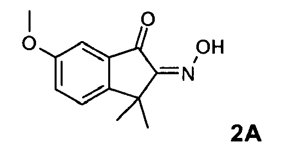
   - Suspendieren der Verbindung **2** in Wasser;
   - Zugabe von einem 2-20 fachen molaren Überschuss an 45%iger Natronlauge, bezogen auf **2**;
   - Erwärmen der Lösung auf 40-60°C;
   - Zugabe von einem 1 bis 2 fachen molaren Überschuss an Benzolsulfonylchlorid, bezogen auf **2**, gegebenenfalls in Toluol gelöst;
   - Rühren der Mischung für 50-70 min;
   - Waschen der wässrige Phase mit Toluol;
   - Ansäuern mit konz. Salzsäure;
   - Waschen der wässrige Phase mit Toluol;
   - Einengen der organischen Phase zur Trockene;
   - Umkristallisieren des Rückstandes aus Isopropanol;

### VERWENDETE BEGRIFFE UND DEFINITIONEN

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden, dem entsprechend werden unter dem Begriff "C₁₋₄Alkyl" verzweigte und unverzweigte Alkylgruppen mit 1 bis 4 Kohlenstoffatomen verstanden. Bevorzugt sind Alkylgruppen mit 1 bis 4 Kohlenstoffatome. Beispielsweise werden hierfür genannt: Methyl, Ethyl, *n*-Propyl, *iso*-Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür genannt: Phenyl oder Naphthyl, bevorzugter Arylrest ist Phenyl. Soweit nicht anders beschreiben, können die Aromaten substituiert sein mit einem oder mehreren Resten ausgewählt aus der Gruppe bestehend aus Methyl, Ethyl, iso-Propyl, tert-Butyl, Hydroxy, Fluor, Chlor, Brom und Jod.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Unter einer "Säure" wird im Rahmen der Erfindung ein Protonendonor verstanden. Dabei kann die Säure in einer wässriger Lösung von unterschiedlichen Konzentrationen vorliegen oder in Reinform (gasförmig, flüssig oder fest) in eine Reaktionslösung eingeleitet werden. Bevorzugt hierbei sind anorganische Säuren, besonders bevorzugt Mineralsäuren. Beispielsweise werden hierfür in konzentrierter oder verdünnter wässriger Lösung genannt: HCl, HNO₃, H₂SO₄, H₂CO₃ oder H₃PO₄.

Unter einer "Lewis-Säure" wird im Rahmen der Erfindung ein Elektronenpaarakzeptor, bevorzugt eine neutrale Verbindung mit einer Elektronenlücke verstanden. Beispielsweise werden hierfür genannt: B(CH₃)₃, BF₃, SO₃, AlCl₃, SiCl₄ oder PF₅

Unter einem "Lösungsmittel" wird im Rahmen der Erfindung ein organsicher, niedermolekularer Stoff verstanden, der andere organische Stoffe auf physikalischem Wege zur Lösung bringen kann. Voraussetzung für die Eignung als Lösungsmittel ist, dass sich beim Lösungsvorgang weder der lösende noch der gelöste Stoff chemisch verändern, dass also die Komponenten der Lösung durch physikalische Trennverfahren wie Destillation, Kristallisation, Sublimation, Verdunstung, Adsorption in der Originalgestalt wiedergewonnen werden können. Aus verschiedenen Gründen können nicht nur die reinen Lösungsmittel, sondern Gemische, die die Lösungseigenschaften vereinigen verwendet werden. Beispielsweise seinen genannt:
- Alkohole, bevorzugt Methanol, Ethanol, Propanole, Butanole, Octanole, Cyclohexanol;
- Glykole, bevorzugt Ethylenglykol, Diethylenglykol;
- Ether / Glykolether, bevorzugt Diethylether, Dibutylether, Anisol, Dioxan, Tetrahydrofuran, Mono-, Di-, Tri-, Polyethylenglykolether;
- Ketone, bevorzugt Aceton, Butanon, Cyclohexanon;
- Ester, bevorzugt Essigsäureester, Glykolester;
- Amide u.a. Stickstoff-Verbindungen, bevorzugt Dimethylformamid, Pyridin, N-Methylpyrrolidon, Acetonitril;
- Schwefel-Verbindungen, bevorzugt Schwefelkohlenstoff, Dimethylsulfoxid, Sulfolan;
- Nitro-Verbindungen bevorzugt Nitrobenzol;
- Halogenkohlenwasserstoffe, bevorzugt Dichlormethan, Chloroform, Tetrachlormethan, Tri-, Tetrachlorethen, 1,2-Dichlorethan, Chlorfluorkohlenstoffe;
- aliphatische oder alicyclische Kohlenwasserstoffe, bevorzugt Benzine, Petrolether, Cyclohexan, Methylcyclohexan, Decalin, Terpen-L.; oder
- aromatische Kohlenwasserstoffe, bevorzugt Benzol, Toluol, Xylole; oder entsprechende Gemische davon. Bevorzugt im Rahmen dieser Erfindung sind:
- Alkohole, bevorzugt Methanol, Ethanol, Propanole, Butanole;
- Ether, bevorzugt Diethylether, Dibutylether, Tetrahydrofuran;
- Ketone, bevorzugt Aceton, Butanon, Cyclohexanon;
- Ester, bevorzugt Essigsäureester;
- Halogenkohlenwasserstoffe, bevorzugt Dichlormethan, Chloroform, Tri-, Tetrachlorethen, 1,2-Dichlorethan;
- aromatische Kohlenwasserstoffe, bevorzugt Benzol, Toluol, Xylole; oder entsprechende Gemische davon. Besonders bevorzugt im Rahmen dieser Erfindung sind Methanol, Ethanol, Propanole, Butanole, Diethylether, Dibutylether, Tetrahydrofuran, Aceton, Cyclohexanon, Essigsäureester, Dichlormethan, Chloroform, 1,2-Dichlorethan, Toluol, Xylole oder entsprechende Gemische davon.

Unter dem Begriff "C₁₋₈-Alkohol" werden verzweigte und unverzweigte Alkohole mit 1 bis 8 Kohlenstoffatomen verstanden und einer oder zwei Hydroxygruppen. Dem entsprechend werden unter dem Begriff "C₁₋₄-Alkohol" verzweigte und unverzweigte Alkylgruppen mit 1 bis 4 Kohlenstoffatomen und einer oder zwei Hydroxygruppen verstanden. Bevorzugt sind Alkohole mit 1 bis 4 Kohlenstoffatome. Beispielsweise werden hierfür genannt: Methanol, Ethanol, *n*-Propanol, *iso*-Propanol, *n*-Butanol, *iso*-Butanol, *sec*-Butanol, *tert*-Butanol, *n-*Pentanol, *iso*-Pentanol, *neo*-Pentanol oder Hexanol. Gegebenenfalls werden für vorstehend genannten Moleküle auch die Abkürzungen MeOH, EtOH, *n*-PrOH, *i*-PrOH, *n-*BuOH, *i*-BuOH, *t*-BuOH, etc. verwendet. Sofern nicht anders beschrieben, umfassen die Definitionen Propanol, Butanol, Pentanol und Hexanol alle denkbaren isomeren Formen der jeweiligen Reste. So umfasst beispielsweise Propanol *n*-Propanol und *iso*-Propanol, Butanol umfasst *iso-*Butanol, *sec*-Butanol und *tert*-Butanol etc.

Die Lösung einer starken Base wird vorzugsweise aus Wasser und 25-75 Gewichts% einer Base gebildet. Als Base können organische Basen oder anorganische Basen, z.B. als wässrige Lösungen verwendet werden. Als anorganische Basen eignen sich basisch reagierende Alkalisalze oder Alkalihydroxide. Bevorzugt werden Alkalihydroxiden eingesetzt. Ganz besonders bevorzugt werden Na₂CO₃, K₂CO₃, LiOH, NaOH, KOH oder NaHCO₃ verwendet. Als organische Basen eignen sich tertiäre Amine, insbesondere tertiäre Alkylamine, tertiäre Alkyl-Arylamine oder Pyridine. Bevorzugt werden Trialkylamine mit verzweigten oder unverzweigten C₁₋₆-Alkylresten eingesetzt. Als ganz besonders bevorzugt haben sich beispielsweise Triethylamin oder Düsopropylethylamin bewährt. Gegebenenfalls kann die Reaktion auch in Gegenwart von basischen Polymeren mit z.B. tertiären Aminofunktionen durchgeführt werden.

Das erfindungsgemäße Verfahren soll nun durch die nachfolgenden Beispiele erläutert werden. Dem Fachmann ist bewusst, dass die Beispiele nur zur Veranschaulichung dienen und als nicht limitierend anzusehen sind.

### BEISPIELE

### VERBINDUNG 3A

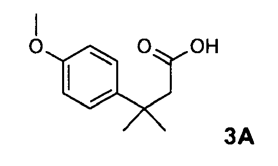

In 11 62%ige Bromwasserstoffsäure werden bei 20°C unter Rühren 250g 3,3-Dimethylacrylsäure eingetragen. Es wird 60 min bei 20°-35°C gerührt, anschließend wird die Mischung bei 35°C mit 900 ml Toluol in mehreren Portionen extrahiert. Die vereinigten organischen Extrakte werden im Vakuum zur Trockene eingeengt. Man erhält 417g (92%) Rohprodukt. 408g des Rohprodukts werden in 660ml Anisol gelöst und im Vakuum werden 70ml Lösungsmittel abdestilliert. Die 35°C warme Lösung wird in eine auf 20°C temperierte Mischung aus 1l Anisol und 442,4g Aluminiumchlorid gegeben. Es wird 4h bei Raumtemperatur nachgerührt, anschließend wird die Lösung in 1,51 Eiswasser gegossen und 80min nachgerührt. Die Phasen werden getrennt und die organische Phase wird mit 250ml 62%iger Bromwasserstoffsäure extrahiert. Danach wird die organische Phase mit 1,51 Natronlauge bei pH 10 - 11 extrahiert. Nach der Phasentrennung wird die wässrige Phase noch 2 mal mit je 350ml Toluol gewaschen. Die wässrige Phase wird bei 50 - 60°C mit Bromwasserstoffsäure auf pH 1 - 2 gestellt und mit 1,251 Toluol in 2 Portionen extrahiert. Diese Toluolphasen werden im Vakuum zur Trockene eingeengt und der Rückstand mit 510ml Methylcyclohexan bei 5°C kristallisiert. Der erhaltene Feststoff wird abgetrennt, mit 100ml Methylcyclohexan gewaschen und im Vakuum bei 50°C getrocknet. Ausbeute 149,8g (32%) der Verbindung **3A**.

### VERBINDUNG 2A

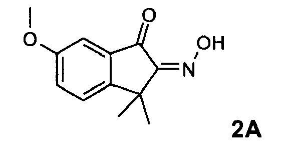

In 1,11 kg Polyphosphorsäure werden 222g der Verbindung **3A** eingetragen. Die Mischung wird auf 70°C erwärmt und 30min bei dieser Temperatur gerührt. Nach dem Abkühlen auf 50°C werden 1,1l Wasser unter Kühlung zugegeben. Die so entstehende Lösung wird mit 430ml Toluol extrahiert. Anschließend wird die Toluolphase mit 220ml Natronlauge bei pH 9 gewaschen und die wässrige Phase mit 80ml Toluol extrahiert. Die vereinigten Toluolphasen werden mit 120ml verdünnter Salzsäure bei etwa pH 3 gewaschen und danach im Vakuum eingeengt. Der verbliebene Rückstand wird in 650ml Methanol gelöst und mit 125ml konz. Salzsäure versetzt. Die Mischung wird auf 50°C erwärmt, portionsweise werden 326g Isoamylnitrit zugegeben und es wird 1h bei 50°C nachgerührt. Nach Abkühlen auf 20°C wird der erhaltene Feststoff abfiltriert, mit 240ml Methanol gewaschen und im Vakuum bei 50°C getrocknet. Ausbeute 142g (61 %) der Verbindung **2A.**

### VERBINDUNG 1A

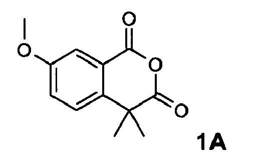

In 370ml Wasser werden 82g von **2A** eingetragen. Es werden 75ml 45%iger Natronlauge zugegeben und die Lösung wird auf 50°C erwärmt. Anschließend wird eine Lösung von 72,7g Benzolsulfonylchlorid in 290ml Toluol unter Rühren zugetropft und es wird 1h bei 50°C nachgerührt. Dann werden die Phasen getrennt und die wässrige Phase wird 2 mal mit je 140ml Toluol gewaschen. Anschließend wird mit 90ml konz. Salzsäure angesäuert und 10min nachgerührt. Danach wird die wässrige Phase mit 300ml und mit 150ml Toluol extrahiert. Die Toluolphasen werden vereinigt und im Vakuum zur Trockene eingeengt. Der Rückstand wird in 360ml Isopropanol in der Siedehitze aufgenommen und nach Abkühlen auf 20°C kristallisiert. Der erhaltene Feststoff wird abgetrennt und im Vakuum bei 50°C getrocknet. Ausbeute 68g (83%) der Verbindung **1A**.

## Patentansprüche

1. Verfahren zur Herstellung einer Verbindung der Formel 1, 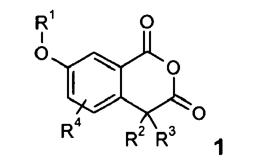 worin R¹, R² und R³ jeweils unabhängig voneinander die Bedeutung von C₁₋₆-Alkyl, C₂₋₆-Alkenyl, C₂₋₆-Alkinyl oder Aryl und R⁴ die Bedeutung von H, OC₁₋₆-Alkyl, OAryl, OH, C₁₋₆-Alkyl, Halogen, CN oder NO₂ hat, **dadurch gekennzeichnet, dass** man
a) eine Acrylsäure der Formel **4** mit einer HBr-Lösung behandelt 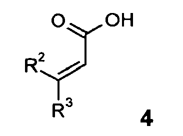 und anschließend mit einer Verbindung der Formel **5** in Gegenwart von Aluminiumchlorid umsetzt; 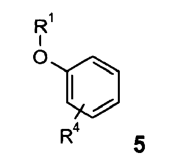
b) die daraus erhaltene Verbindung **3**, 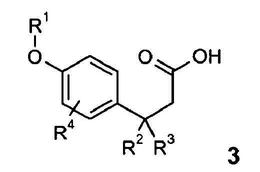 mit Polyphosphorsäure, Mischungen aus Phosphorpentoxid und Methansulfonsäure oder einer Lewis-Säure wie zum Bsp. Wismut- oder einem Lanthanidtriflat oder-bistrifluormethansulfonylimid behandelt oder die Verbindung **3** zunächst in ein Säurehalogenid oder -anhydrid überführt und anschließend mit einer Lewis Säure behandelt und anschließend mit einem C₁₋₆-Alkylnitrit, umsetzt
c) die daraus erhaltene Verbindung **2**, 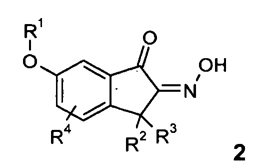 in Gegenwart einer Base mit einem Sulfonsäurehalogenid, bevorzugt Toluolsulfonsäurechlorid umsetzt, verseift und anschließend hydrolisiert.

2. Verfahren nach Anspruch 1 worin R¹, R² und R³ jeweils unabhängig voneinander die Bedeutung von C₁₋₆-Alkyl hat.

3. Verfahren nach Anspruch 1 oder 2, worin R¹, R² und R³ jeweils unabhängig voneinander Methyl, Ethyl, *iso*-Propyl oder *tert-*Butyl Methyl bedeuten.

4. Verfahren nach einem der Ansprüche 1 bis 3, worin R⁴ die Bedeutung von H, Methyl, Ethyl, *iso*-Propyl oder *tert*-Butyl.

5. Verfahren nach einem der Ansprüche 1 bis 4, worin in Schritt a)
• die Acrylsäure der Formel **4** mit einer 40-80%-iger HBr-Lösung behandelt wird,
• die Mischung für 20-100 min bei einer Temperatur von 0-50°C gerührt wird;
• die Mischung mit einem Lösungsmittel extrahiert wird;
• das Lösungsmittel weitestgehend oder ganz abgezogen wird;
• das Zwischenprodukt in einer Verbindung der Formel **5** mit oder ohne Zugabe eines weiteren Lösungsmittels gelöst wird;
• die Mischung nach Zugabe von Aluminiumchlorid für 2-6 Stunden bei 0-50°C gerührt wird;
• nach Beenden der Reaktion das Produkt durch Extraktion und Umkristallisieren gereinigt wird.

6. Verfahren nach einem der Ansprüche 1 bis 5, worin in Schritt a)
• die Acrylsäure der Formel **4** mit einer 60-65%-iger HBr-Lösung behandelt wird,
• die Mischung für 25-70 min bei einer Temperatur von 20-35°C gerührt wird;
• die Mischung mit einem aromatischen Lösungsmittel extrahiert wird;
• das Lösungsmittel weitestgehend oder ganz abgezogen wird;
• das Zwischenprodukt in einer Verbindung der Formel **5** gelöst wird;
• die Mischung nach Zugabe von Aluminiumchlorid für 3-5 Stunden bei 20-30°C gerührt wird;
• die Reaktion durch Zugabe von Wasser beendet wird;
• das Produkt aus der organischen Phase mit der Lösung einer starken Base extrahiert wird,
• die wässrige Phase angesäuert und das Produkt mit einem Lösungsmittel extrahiert wird.
• das Lösungsmittel der organischen Phase weitestgehend oder ganz abgezogen wird;
• der Rückstand aus einem aliphatischen Kohlenwasserstoff umkristallisiert wird;

7. Verfahren nach einem der Ansprüche 1 bis 6, worin in Schritt b)
• die erhaltenen Verbindung **3**, mit Polyphosphorsäure vermischt wird;
• die Mischung für 10-50 min auf 50-90°C erwärmt wird;
• die Mischung mit einem Lösungsmittel extrahiert wird;
• die organische Phase gewaschen wird;
• das Lösungsmittel weitestgehend oder ganz abgezogen wird;
• der Rückstand in einem Alkohol gelöst und mit konz. Salzsäure versetzt wird;
• zu der Mischung ein C₁₋₆-Alklynirit gegeben wird;
• die Mischung für 30-90 min bei 30-70°C gerührt wird;
• der entstandene Feststoff filtriert und gewaschen wird.

8. Verfahren nach einem der Ansprüche 1 bis 7, worin in Schritt b)
• die erhaltenen Verbindung 3, mit Polyphosphorsäure vermischt wird;
• die Mischung für 20-40 min auf 60-80°C erwärmt wird;
• zu der Mischung Wasser gegeben wird;
• die Mischung mit einem Lösungsmittel extrahiert wird
• die organische Phase mit der Lösung einer starken Base bei pH 8.5-9.5 gewaschen wird;
• die organische Phase mit verdünnter Salzsäure gewaschen wird;
• das Lösungsmittel der organischen Phase weitestgehend oder ganz abgezogen wird;
• der Rückstand in einem Alkohol gelöst und mit konz. Salzsäure versetzt wird;
• zu der Mischung Isoamylnitrit gegeben wird;
• die Mischung für 50-70 min bei 40-60°C gerührt wird;
• der entstandene Feststoff filtriert und gewaschen wird.

9. Verfahren nach einem der Ansprüche 1 bis 8, worin in Schritt c)
• die erhaltenen Verbindung **2** in Wasser suspendiert wird;
• die Lösung einer starken Base zugegeben wird;
• die Mischung auf 30-70°C erwärmt wird;
• zu der Mischung Benzolsulfonylchlorid gegeben wird;
• die Mischung für 30-90 min gerührt wird;
• die Mischung mit einem aromatischen Lösungsmittel gewaschen wird;
• die Mischung mit einer Säure angesäuert wird;
• die Mischung mit einem aromatischen Lösungsmittel extrahiert wird;
• das Lösungsmittel der organischen Phase weitestgehend oder ganz abgezogen wird;
• der Rückstand aus einem C₁₋₈-Alkohol umkristallisiert wird.

10. Verfahren nach einem der Ansprüche 1 bis 9, worin in Schritt c)
• die erhaltenen Verbindung **2** in Wasser suspendiert wird;
• die Lösung einer starken Base zugegeben wird;
• die Mischung auf 40-60°C erwärmt wird;
• zu der Mischung Benzolsulfonylchlorid gegeben wird;
• die Mischung für 50-70 min gerührt wird;
• die Mischung mit einem aromatischen Lösungsmittel gewaschen wird;
• die Mischung angesäuert wird mit konz. Salzsäure;
• die Mischung mit einem aromatischen Lösungsmittel extrahiert wird;
• das Lösungsmittel der organischen Phase weitestgehend oder ganz abgezogen wird;
• der Rückstand aus einem C₁₋₈-Alkohol umkristallisiert wird.

## Claims

1. Process for preparing a compound of formula **1**, 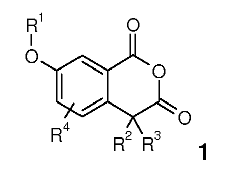 wherein R¹, R² and R³ in each case independently of one another denote C₁₋₆-alkyl, C₂₋₆-alkenyl, C₂₋₆-alkynyl or aryl and R⁴ denotes H, OC₁₋₆-alkyl, Oaryl, OH, C₁₋₆-alkyl, halogen, CN or NO₂, **characterised in that**
a) an acrylic acid of formula **4** is treated with an HBr solution 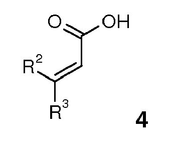 and then reacted with a compound of formula **5** in the presence of aluminium chloride; 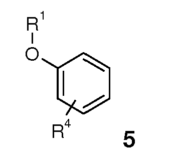
b) the compound **3** obtained therefrom, 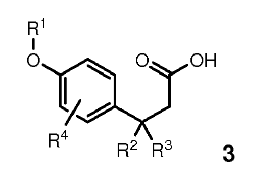 is treated with polyphosphoric acid, mixtures of phosphorus pentoxide and methanesulphonic acid or a Lewis acid such as for example bismuth or a lanthanide triflate or bistrifluoromethanesulphonylimide, or the compound 3 is first of all converted into an acid halide or anhydride and then treated with a Lewis acid and then reacted with a C₁₋₆-alkyl nitrite,
c) the compound 2 obtained therefrom, 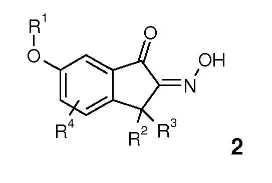 is reacted in the presence of a base with a sulphonic acid halide, preferably toluenesulphonic acid chloride, saponified and then hydrolysed.

2. Process according to claim 1 wherein R¹, R² and R³ in each case independently of one another denote C₁₋₆-alkyl.

3. Process according to claim 1 or 2, wherein R¹, R² and R³ in each case independently of one another denote methyl, ethyl, *iso*-propyl or *tert*-butyl methyl.

4. Process according to one of claims 1 to 3, wherein R⁴ denotes H, methyl, ethyl, *iso*-propyl or *tert*-butyl.

5. Process according to one of claims 1 to 4, wherein in step a)
• the acrylic acid of formula **4** is treated with a 40-80% HBr solution,
• the mixture is stirred for 20-100 min at a temperature of 0-50°C;
• the mixture is extracted with a solvent;
• the solvent is substantially or totally eliminated;
• the intermediate product is dissolved in a compound of formula 5 with or without the addition of another solvent;
• after the addition of aluminium chloride the mixture is stirred for 2-6 hours at 0-50 °C;
• after the end of the reaction the product is purified by extraction and recrystallisation.

6. Process according to one of claims 1 to 5, wherein in step a)
• the acrylic acid of formula **4** is treated with a 60-65% HBr solution,
• the mixture is stirred for 25-70 min at a temperature of 20-35°C;
• the mixture is extracted with an aromatic solvent;
• the solvent is substantially or totally eliminated;
• the intermediate product is dissolved in a compound of formula **5**;
• after the addition of aluminium chloride the mixture is stirred for 3-5 hours at 20-30 °C;
• the reaction is ended by the addition of water;
• the product is extracted from the organic phase with the solution of a strong base,
• the aqueous phase is acidified and the product is extracted with a solvent.
• the solvent of the organic phase is substantially or totally eliminated;
• the residue is recrystallised from an aliphatic hydrocarbon.

7. Process according to one of claims 1 to 6, wherein in step b)
• the compound 3 obtained is mixed with polyphosphoric acid;
• the mixture is heated for 10-50 min to 50-90°C;
• the mixture is extracted with a solvent;
• the organic phase is washed;
• the solvent is substantially or totally eliminated;
• the residue is dissolved in an alcohol and combined with conc. hydrochloric acid;
• a C₁₋₆-alkyl nitrite is added to the mixture;
• the mixture is stirred for 30-90 min at 30-70°C;
• the resulting solid is filtered and washed.

8. Process according to one of claims 1 to 7, wherein in step b)
• the compound 3 obtained is mixed with polyphosphoric acid;
• the mixture is heated for 20-40 min to 60-80°C;
• water is added to the mixture;
• the mixture is extracted with a solvent
• the organic phase is washed with the solution of a strong base at pH 8.5-9.5;
• the organic phase is washed with dilute hydrochloric acid;
• the solvent of the organic phase is substantially or totally eliminated;
• the residue is dissolved in an alcohol and combined with conc. hydrochloric acid;
• isoamyl nitrite is added to the mixture;
• the mixture is stirred for 50-70 min at 40-60°C;
• the resulting solid is filtered and washed.

9. Process according to one of claims 1 to 8, wherein in step c)
• the compound 2 obtained is suspended in water;
• the solution of a strong base is added;
• the mixture is heated to 30-70°C;
• benzenesulphonyl chloride is added to the mixture;
• the mixture is stirred for 30-90 min;
• the mixture is washed with an aromatic solvent;
• the mixture is acidified with an acid;
• the mixture is extracted with an aromatic solvent;
• the solvent of the organic phase is substantially or totally eliminated;
• the residue is recrystallised from a C₁₋₈-alcohol.

10. Process according to one of claims 1 to 9, wherein in step c)
• the compound 2 obtained is suspended in water;
• the solution of a strong base is added;
• the mixture is heated to 40-60°C;
• benzenesulphonyl chloride is added to the mixture;
• the mixture is stirred for 50-70 min;
• the mixture is washed with an aromatic solvent;
• the mixture is acidified with conc. hydrochloric acid;
• the mixture is extracted with an aromatic solvent;
• the solvent of the organic phase is substantially or totally eliminated;
• the residue is recrystallised from a C₁₋₈-alcohol.

## Revendications

1. Procédé de production d'un composé de formule 1, 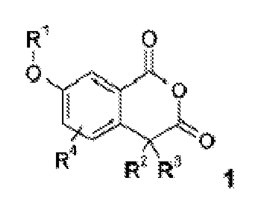 dans laquelle R¹, R² et R³ représentent respectivement, indépendamment les uns des autres, un groupe alkyle en C₁ à C₆, alcényle en C₂ à C₆, alcinyle en C₂ à C₆ ou aryle et R⁴ représente H, un groupe O-alkyle en C₁ à C₆, O-aryle, OH, alkyle en C₁ à C₆, un atome d'halogène, CN ou NO₂, **caractérisé en ce que** l'on traite
a) un acide acrylique de formule 4 avec une solution de HBr 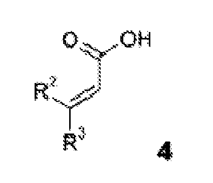 et ensuite, on fait réagir avec un composé de formule 5 en présence de chlorure d'aluminium ; 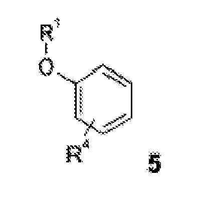
b) on traite le composé 3 ainsi obtenu 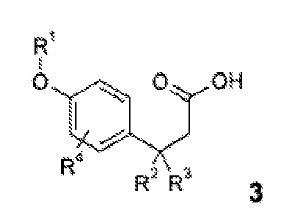 avec de l'acide polyphosphorique, des mélanges de pentoxyde de phosphore et de l'acide méthanesulfonique ou un acide de Lewis tel que, par exemple, du triflate de bismuth ou un triflate de lanthanide ou du bistrifluorométhane-sulfonylimide de bismuth ou du bistrifluorométhane-sulfonylimide de lanthanide ou le composé 3 est tout d'abord converti en un halogénure d'acide ou anhydride d'acide et ensuite, on traite avec un acide de Lewis, puis, on fait réagir avec un nitrite d' alkyle en C₁ à C₆,
c) on fait réagir le composé 2 ainsi obtenu 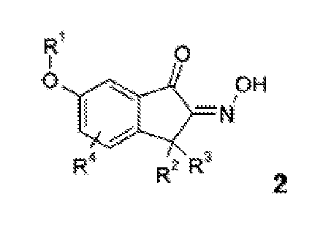 en présence d'une base avec un halogénure d'acide sulfonique, de préférence du chlorure d'acide toluènesulfonique, on le saponifie et ensuite, on l'hydrolyse.

2. Procédé selon la revendication 1, dans lequel R¹, R² et R³ désignent respectivement, indépendamment les uns des autres, un groupe alkyle en C₁ à C₆.

3. Procédé selon la revendication 1 ou 2, dans laquelle R¹, R² et R³ représentent respectivement, indépendamment les uns des autres, un groupe méthyle, éthyle, iso-propyle ou tert-butyle-méthyle.

4. Procédé selon l'une des revendications 1 à 3, dans lequel R⁴ représente H, un groupe méthyle, éthyle, iso-propyle ou tert-butyle.

5. Procédé selon l'une des revendications 1 à 4, dans lequel, à l'étape a)
- l'acide acrylique de formule 4 est traité avec une solution de HBr de 40 à 80 %,
- le mélange est agité pendant 20 à 100 min à une température de 0 à 50° C ;
- le mélange est extrait avec un solvant ;
- le solvant est retiré largement ou totalement ;
- le produit intermédiaire est dissous dans un composé de formule 5 avec ou sans addition d'un autre solvant ;
- le mélange est agité après addition de chlorure d'aluminium pendant 2 à 6 heures de 0 à 50° C ;
- après la fin de la réaction, le produit est purifié par extraction et recristallisation.

6. Procédé selon l'une des revendications 1 à 5, dans lequel, à l'étape a)
- l'acide acrylique de formule 4 est traité avec une solution de HBr de 60 à 65 %,
- le mélange est agité pendant 25 à 70 min à une température de 20 à 35° C ;
- le mélange est extrait avec un solvant aromatique ;
- le solvant est retiré largement ou totalement ;
- le produit intermédiaire est dissous dans un composé de formule 5 ;
- le mélange est agité après addition de chlorure d'aluminium pendant 3 à 5 heures de 20 à 30° C ;
- la réaction est terminée par addition d'eau ;
- le produit de la phase organique est extrait avec la solution d'une base forte,
- la phase aqueuse est acidifiée et le produit est extrait avec un solvant,
- le solvant est largement ou totalement retiré de la phase organique,
- le résidu est recristallisé à partir d'un hydrocarbure aliphatique.

7. Procédé selon l'une des revendications 1 à 6, dans lequel à l'étape b)
- le composé 3 obtenu est mélangé avec de l'acide polyphosphorique ;
- le mélange est chauffé pendant 10 à 50 min de 50 à 90° C ;
- le mélange est extrait avec un solvant ;
- la phase organique est lavée ;
- le solvant est largement ou complètement éliminé ;
- le résidu est dissous avec un alcool et est mélangé avec de l'acide chlorhydrique concentré ;
- on ajoute au mélange du nitrite d'alkyle en C₁ à C₆ ;
- le mélange est agité pendant 30 à 90 min de 30 à 70° C ;
- le solide obtenu est filtré et lavé.

8. Procédé selon l'une des revendications 1 à 7, dans lequel, à l'étape b)
- le composé 3 obtenu est mélangé avec de l'acide polyphosphorique ;
- le mélange est chauffé pendant 20 à 40 min de 60 à 80° C ;
- on ajoute de l'eau au mélange ;
- le mélange est extrait avec un solvant ;
- la phase organique est lavée avec la solution d'une base forte à pH 8,5 à 9,5 ;
- la phase organique est lavée avec de l'acide chlorhydrique dilué ;
- le solvant est largement ou complètement retiré de la phase organique ;
- le résidu est dissous dans un alcool et mélangé avec de l'acide chlorhydrique concentré ;
- au mélange, on ajoute de l'isoamylnitrite ;
- le mélange est agité pendant 50 à 70 min de 40 à 60° C ;
- le solide obtenu est filtré et lavé.

9. Procédé selon l'une des revendications 1 à 8, dans lequel à l'étape c)
- le composé 2 obtenu est mis en suspension dans de l'eau ;
- la solution est ajoutée à une base forte ;
- le mélange est chauffé de 30 à 70° C ;
- au mélange, on ajoute du chlorure de benzènesulfonyle ;
- le mélange est agité pendant 30 à 90 min ;
- le mélange est lavé avec une solution aromatique ;
- le mélange est acidifié avec un acide ;
- le mélange est extrait avec un solvant aromatique ;
- le solvant est largement ou complètement retiré de la phase organique ;
- le résidu est recristallisé à partir d'un alcool en C₁ à C₈.

10. Procédé selon l'une des revendications 1 à 9, dans lequel à l'étape c)
- le composé 2 obtenu est mis en suspension dans de l'eau ;
- la solution est ajoutée à une base forte ;
- le mélange est chauffé de 40 à 60° C ;
- on ajoute au mélange du chlorure de benzènesulfonyle ;
- le mélange est agité pendant 50 à 70 min ;
- le mélange est lavé avec un solvant aromatique ;
- le mélange est acidifié avec de l'acide chlorhydrique concentré ;
- le mélange est extrait avec un solvant aromatique ;
- le solvant est largement ou complètement retiré de la phase organique ;
- le résidu est recristallisé à partir d'un alcool en C₁ à C₈.
